# EUROPEAN PATENT APPLICATION

(11) **EP 2 258 878 A1**
(43) Date of publication of application: **08.12.2010**
(21) Application number: 10160825.5
(22) Date of filing: 23.04.2010
(51) Int. Cl.: C12Q 1/70

(54) **A method for detecting HBV using real time PCR**

(30) Priority: 23.04.2009 KR 20090035638
(71) Applicant: LG Life Sciences, Ltd., Seoul 150-721 (KR)
(72) Inventor: Lee, Dong Hwan, Daejeon 305-380 (KR); Kang, Jin Seok, Daejeon 305-380 (KR); Park, Young Suk, Daejeon 305-380 (KR); Kim, Dong Hyun, Daejeon 305-380 (KR)
(74) Representative: Bassil, Nicholas Charles

(57) **Abstract**

The present invention relates to the detection of hepatitis B virus (HBV) gene. More particularly, the present invention relates to a pair of primers complementary to HBV gene, a composition for the detection of HBV comprising a pair of primers and a probe complementary to HBV gene, a HBV detection kit comprising the composition, and a method for detecting HBV gene.

<110>	LG LIFE SCIENCES LTD.

<120>	A METHOD FOR DETECTING HBV USING REAL TIME PCR

<130>	P49565EP

<150>	KR10-2009-0035638
<151>	2009-04-23

<160>	9

<170>	KopatentIn 1.71

<210>	1
<211>	20
<212>	DNA
<213>	Artificial Sequence

<220>
<223>	forward primer for amplication of HBV S protein

<400>	1
gtrtctgcgg cgttytatca

20

<210>	2
<211>	20
<212>	DNA
<213>	Artificial Sequence

<220>
<223>	Reverse primer for amplication of HBV S protein region

<400>	2
ggacaaacgg gcaacatacc

20

<210>	3
<211>	24
<212>	DNA
<213>	Artificial Sequence

<220>
<223>	forward primer for amplication of HBV pre-Core region

<400>	3
aaatgcccct atcytatcaa cact

24

<210>	4
<211>	22
<212>	DNA
<213>	Artificial Sequence

<220>
<223>	Reverse primer for amplication of HBV pre-Core region

<400>	4
ccgagattka gatcttctgc ga

22

<210>	5
<211>	25
<212>	DNA
<213>	Artificial Sequence

<220>
<223>	forward primer for amplication of GFP

<400>	5
cgatggccct gtccttttac attta

25

<210>	6
<211>	25
<212>	DNA
<213>	Artificial Sequence

<220>
<223>	Reverse primer for amplication of GFP

<400>	6
cttttcgttg ggatctttga ttaaa

25

<210>	7
<211>	25
<212>	DNA
<213>	Artificial Sequence

<220>
<223>	probe for HBV S protein region

<400>	7
catcctgctg ctatgcctca tcttc

25

<210>	8
<211>	26
<212>	DNA
<213>	Artificial Sequence

<220>
<223>	probe for HBV pre-Core region

<400>	8
cccctagaag aaagaactcc ctcgcc

26

<210>	9
<211>	26
<212>	DNA
<213>	Artificial Sequence

<220>
<223>	probe for GFP

<400> 9
attacctgtc cacacaatct gccctt

26

## Description

### [Technical Field]

The present invention relates to the detection of hepatitis B virus gene. More particularly, the present invention relates to a pair of primers complementary to HBV gene, a composition for the detection of HBV comprising a pair of primers and a probe complementary to HBV gene, a HBV detection kit comprising the composition, and a method for detecting HBV gene.

### [Background Art]

Hepatitis B Virus (hereinbelow, referred to as HBV) is known as a major cause of viral hepatitis, and an estimated 2 billion people are infected with HBV worldwide. Of these infected people, approximately 350 million people are chronic carriers, and a majority of the people are at substantially increased risk of developing liver cirrhosis, and hepatocellular carcinoma. The HBV envelope gene consists of the preS region composed of preS1 and preS2, and the S region. After transcription of the entire envelope gene, three envelope proteins (L protein, M protein and S protein) are synthesized by alternate translation at each of three regions. The L protein, for which translation is initiated at the preS1 region, consists of approximately 400 amino acids containing all of the preS1 domain, preS2 domain and S domain. The M protein, for which translation is initiated at the preS2 region, consists of approximately 281 amino acids containing the preS2 domain and S domain. The S protein consists of approximately 226 amino acids containing the S domain only, and is the most abundantly expressed to be the main component of virus particles. The S domains of the envelope protein bind to each other to form a particle as S antigen. The preS1 and preS2 domains contained in the M protein and L protein are located on the outside of the particle, and serve as a preS antigen to induce high immune responses.

Diagnosis of HBV infection can be performed by detecting an antibody against HBV that is present in a sample, detecting an HBV antigen, or detecting an HBV gene.

Frequently, the method of HBV antigen detection is to detect its absence or presence in blood using HBV Surface Antigen (HBsAg) or HBV e antigen (HBeAg) as a diagnostic marker. However, early diagnosis of HBV infection can be hindered depending on the life cycle of the virus. Reduction or absence of HBsAg or HBeAg in the blood of an HBV-infected patient is also an obstacle to an accurate diagnosis of HBV in a patient during treatment.

HBV gene detection is performed by a nucleic acid amplification technique (NPT) or a DNA probe method. At present, these methods are widely employed in clinical practice. In particular, PCR is a method to selectively amplify a low amount of a target gene to a detectable level, and thus it is widely used. However, there is a problem in that the DNA extraction procedures lead to an inevitable loss of DNA. For example, even though a sufficient amount of the target DNA is contained in the sample as a template for amplification, the suitable amount of DNA for amplification could not be recovered due to the loss during the extraction process, leading to a reduction in the amplification efficiency of the template. Therefore, it is hard to sufficiently amplify the target DNA and detect the target DNA in the sample. Consequently, the technique produces a false negative result, and thus it is difficult to ensure accurate results. Even though using the same sample, variations in DNA yields during the extraction process may produce different results, leading to a lack of reproducibility. That is, the DNA amplification technique has problems in that it requires the DNA extraction process to cause the loss of trace target DNA in the sample, whereby the detection sensitivity is reduced. In addition, when amplification inhibitors (e.g., heparin, surfactant, protein denaturants, organic solvent, etc.) are contained in the sample liquid, the amplification efficiency is also reduced to lower the detection sensitivity.

Further, a region having very little genetic variation, such as the S protein, the pre-Core region or the envelope protein, is useful for the HBV detection by PCR. Nevertheless, these regions also have a low probability of variation such as mutation. If genetic variation in the base sequence occurs, the amplification procedures using a PCR primer and/or probe are failed. Consequently, a false-negative result is obtained or the detection sensitivity is reduced due to reduced amplification efficiency. Thus, it is difficult to ensure accurate quantification.

### [Disclosure]

### [Technical Problem]

The present inventors have made many efforts to solve these problems. They have developed a method of accurately and rapidly detecting and quantifying HBV gene using a trace amount of target HBV gene contained in a sample, and a method for simultaneous detection and quantification of two regions of HBV gene having very little genetic variation such as mutation. According to these methods, even though genetic variation via mutation occurs in one region of the gene, the other region of the gene can be detected, so as to achieve accurate diagnosis of patients with suspected HBV infection with high sensitivity and specificity, thereby completing the present invention.

### [Technical Solution]

It is an object of the present invention to provide a pair of primers complementary to HBV gene, which have base sequences represented by SEQ ID NOs. 1 and 2, or SEQ ID NOs. 3 and 4.

It is another object of the present invention to provide a composition for the detection of HBV gene, comprising a pair of primers having base sequences of SEQ ID NOs. 1 and 2, a pair of primers having base sequences of SEQ ID NOs. 3 and 4, and probes having base sequences represented by SEQ ID NOs. 7 and 8.

It is still another object of the present invention to provide an HBV detection kit comprising the composition for the detection of HBV gene.

It is still another object of the present invention to provide a method for detecting HBV gene using the composition for the detection of HBV gene.

### [Description of Drawings]

FIG. 1 is the result showing that a positive detection and quantification can be made without competitive inhibition of the internal control for the HBV-specific primer and probe;
FIG. 2 shows a linearity of HBV quantification standard;
FIG. 3 shows the result of PCR, in which the amplification of HBV S-protein and pre-core region was performed separately; and
FIG. 4 shows the result of PCR, in which the amplification of HBV S-protein and the pre-core region was performed simultaneously. In this case, there was also no competitive inhibition in the detection and no significant difference in the quantification.

### [Advantageous Effects]

As described above, when the detection and quantification of HBV infection is performed by the Real-Time PCR according to the present invention, the sensitivity and specificity equal to or greater than that of the known methods can be obtained.

### [Best Mode]

Accordingly, in accordance with one aspect, the present invention relates to a pair of primers complementary to HBV gene. Preferably, the primers are a pair of primers having the base sequences represented by SEQ ID NOs. 1 and 2 or SEQ TD NOs. 3 and 4.

As used herein, the term "primer" means a short nucleic acid sequence having the free 3' hydroxyl group, which forms a base-pair with a complementary template and serves as a starting point for template strand replication. The primer is able to initiate DNA synthesis in the presence of four different nucleoside triphosphates and an agent for polymerization in an appropriate buffer and at a suitable temperature. In accordance with the object of the present invention, the primer is a primer that specifically amplifies the HBV S protein or HBV pre-Core region. Therefore, the primer of the present invention is composed of a pair of forward and reverse primers, which are complementary to the gene and have base sequences of 7 to 50, preferably 10 to 30. In particular, the HBV S gene region can be specifically amplified by a pair of primers having the base sequences of SEQ ID NOs. 1 and 2, and the HBV pre-Core region can be specifically amplified by a pair of primers having the base sequences of SEQ ID NOs. 3 and 4.

The primer of the present invention may be chemically synthesized by the phosphoramidite solid support method or other well-known methods. These nucleic acid sequences may also be modified using many means known in the art. Nonlimiting examples of such modifications include methylation, capsulation, replacement of one or more native nucleotides with analogues thereof, and inter-nucleotide modifications, for example, modifications to uncharged conjugates (e.g., methyl phosphonate, phosphotriester, phosphoroamidate, carbamate, etc.) or charged conjugates (e.g., phosphorothioate, phosphorodithioate, etc.). Nucleic acids may contain one or more additionally covalent-bonded residues, which are exemplified by proteins (e.g., nucleases, toxins, antibodies, signal peptides, poly-L-lysine, etc.), intercalating agents (e.g., acridine, proralene, etc.), chelating agents (e.g., metals, radioactive metals, iron, oxidative metals, etc.), and alkylating agents. The nucleic acid sequences of the present invention may also be altered using a label capable of directly or indirectly supplying a detectable signal. Examples of the label include radioisotopes, fluorescent molecules and biotin.

When PCR is performed using the primers having the base sequences of SEQ ID NOs. 1 and 2, and SEQ ID NOs. 3 and 4 provided by the present invention, each HBV genotype can be detected with high specificity and sensitivity. Herein, the term 'sensitivity' means the proportion of people with a disease who tested positive, namely, the ability of a test to recognize the presence of a particular disease in a patient. The term 'specificity' means the proportion of those free of the disease who tested negative, namely, the ability of a test to recognize healthy individuals.

Therefore, the primers can be used in the diagnosis of HBV infection, analysis of infected HBV genotype, epidemiological analysis of HBV, and evaluation of efficacy and safety of HBV vaccine.

In accordance with still another embodiment, the present invention relates to a composition for the detection of HBV gene, comprising a pair of primers having the base sequences of SEQ ID NOs. 1 and 2, a pair of primers having the base sequences of SEQ ID NOs. 3 and 4, and probes having the base sequences of SEQ ID NOs. 7 and 8.

Specifically, the present invention provides a composition for the detection of HBV, comprising novel primers specific to the HBV S protein region, which consist of a sense primer having the base sequence of SEQ ID NO. 1 and/or an antisense primer having the base sequence of SEQ ID NO. 2; and a probe having the base sequence of SEQ ID NO. 7. Further, the present invention provides a composition for the detection of HBV, comprising novel primers specific to the HBV pre-Core region, which consist of a sense primer having the base sequence of SEQ ID NO. 3 and/or an antisense primer having the base sequence of SEQ ID NO. 4; and a probe having the base sequence of SEQ ID NO. 8.

Further, the present invention provides a composition for the detection of an internal control, comprising novel primers that are specific to a gene being not present in human and HBV gene and serves as an internal control to examine a false negative reaction, in which the primers consist of a sense primer having the base sequence of SEQ ID NO. 5 and/or an antisense primer having the base sequence of SEQ ID NO. 6; and a probe having the base sequence of SEQ ID NO. 9.

In the present invention, the detection of HBV in blood can be preferably achieved with higher sensitivity and specificity by using the S protein-specific primer and probe together with the pre-Core-specific primer and probe, compared to the technique using a primer and probe specific to base sequence of a single gene. The specific sequences of the primers are described in the following Table 1.

**[Table 1]**

| Name | Primer base sequence | Length | SEQ ID No. |
|---|---|---|---|
| BSFOR | GTR TCT GCG GCG TTY TAT CA | 20 | 1 |
| BSREV | GGA CAA ACG GGC AAC ATA CC | 20 | 2 |
| BCFOR | AAA TGC CCC TAT CYT ATC AAC ACT | 24 | 3 |
| BCREV | CCG AGA TTK AGA TCT TCT GCG A | 22 | 4 |
| ICFOR | CGA TGG CCC TGT CCT TTT ACA TTT A | 25 | 5 |
| ICREV | CTT TTC GTT GGG ATC TTT GAT TAA A | 25 | 6 |

As used herein, the term "HBV (Hepatitis B virus)" is a kind of virus, and causes hepatitis B that is commonly transmitted by blood transfusion. It is largely divided into acute and chronic hepatitis. The symptoms of acute hepatitis B are similar to those of hepatitis A or C. Most people have no symptoms, or some people experience flu-like symptoms, but recover without any treatment. The symptoms of chronic hepatitis B are also similar to those of hepatitis C, and include fatigue, fever, vomiting, muscle and joint pain. The symptoms continue for long periods of time, increasing the risk of development of hepatic cirrhosis and hepatoma. Therefore, continuous examination is required. PCR is generally used for diagnosis of hepatitis B, but the known PCR diagnostic methods are problematic in that the methods amplify and detect a single region, and thus mutation in the region generates low sensitivity and specificity, thereby providing false positive and false negative results. In the diagnostic method of the present invention, the S protein-specific primer and probe are used together with the pre-Core region-specific primer and probe, and their amplification products are used in a single detection step, thereby providing convenience, high specificity and sensitivity, and improved accuracy of quantitative determination.

In the preferred embodiment, the composition of the present invention further comprises a probe sequence to detect the nucleic acids that are amplified by the primer sequences.

As used herein, the term "probe" refers to an oligonucleotide, whether occurring naturally or produced synthetically, recombinantly or by PCR amplification, which is capable of hybridizing to another oligonucleotide of interest. A probe may be single-stranded or double-stranded. Probes are useful in the detection, identification and isolation of particular gene sequences. In the preferred embodiment, it is contemplated that any probe used in the present invention will be labeled with any "reporter molecule," so that is detectable in any detection system including fluorescent, radioactive, and luminescent systems. It is not intended that the present invention is limited to any particular detection system or label. Preferably, the probe of the present invention is a probe represented by SEQ ID NO. 7 or 8. In the preferred Example of the present invention, it was found that the primer and probe were used to detect the presence and absence of HBV with high sensitivity and specificity and there was no cross-reactivity.

In one preferred embodiment, the probe of the present invention may be a probe having fluorescent molecules at its 5' and 3' ends. In one preferred embodiment, the probe contains a fluorescent reporter and a quencher at its 5' and 3' ends, respectively, in which they may show interference with each other. Therefore, when the probes bind to the S protein or pre-Core region present in the sample, the generation of fluorescence signals is restricted. Upon performing polymerase chain reaction, the probe is decomposed, and the fluorescent reporter at 5'end is released away from the quencher at 3' end, thereby generates fluorescence signals. The presence of HBV in the sample can be detected by the fluorescence signals.

Any fluorescent molecules that are typically used in the related art can be labeled at 5' end, exemplified by 6-carboxyfluorescein, hexachloro-6-carboxyfluorescein, tetrachloro-6-carboxyfluorescein, FAM (5-carboxy fluorescein), HEX (2',4',5',7'-tetrachloro-6-carboxy-4,7-dichlorofluorescein) and Cy5 (cyanine-5), but are not limited thereto.

Any fluorescent molecules that are typically used in the related art can be also labeled at 3' end without limitation, exemplified by 6-carboxytetramethyl-rhodamine, TAMRA (5-Carboxytetramethylrhodamine), and BHQ 1, 2 and 3 (black hole quencher 1, 2, 3), but are not limited thereto.

In the present invention, base sequences of the probes of SEQ ID NOs. 7 to 9 are described in the following Table 2.

**[Table 2]**

| **Name** | **5'** | **Probe base sequence (5'- >3')** | **3'** | **Length** | **SEQ ID No .** |
|---|---|---|---|---|---|
| BSFAM | FAM | CAT CCT GCT GCT ATG CCT CAT CTT C | TAMRA | 25 | 7 |
| BCFAM | FAM | CCC CTA GAA GAA AGA ACT CCC TCG CC | TAMRA | 26 | 8 |
| ICCy5 | Cy5 | ATT ACC TGT CCA CAC AAT CTG CCC TT | BHQ3 | 26 | 9 |

The HBV-specific primer and probe of the present invention are able to produce the PCR product having a length of 100 bp, which is suitable for Real Time PCR. In particular, the probe specific to S-protein and pre-Core is useful for PCR quantification analysis by taqman assay or molecular beacon assay.

The present inventors have examined the risk of false positive and false negative results by base sequence analysis. They found that the primer and probe of the present invention is a primer base sequence capable of amplifying HBV gene only, and shows a 100% positive predictive value in the test using Genotype Panel ((PHD201(M)) provided by BBI, indicating very low risk of false positive and false negative results. In addition, their quantitative results were evaluated using Genotype Panel. As a result, there was no significant difference in the quantitative results between panels (Table 5). Accordingly, the primers of SEQ ID NOs. 1 and 2, and the probes of SEQ ID NOs. 3 and 4 and SEQ ID NOs. 7 and 8 are used to perform the detection and quantitation of HBV in blood with high reliability.

In the preferred embodiment, the present invention may include additional primer and probe sequences as an internal control to minimize the false positive and false negative results. The internal control group serves as a marker for direct comparison to prevent false positive and false negative results.

Preferably, the internal control group may be a GFP (Green Fluorescence Protein) derived from a plant. More preferably, the GFP primer sequence may include the primers represented by SEQ ID NOs. 5 and 6 (see Table 1).

The primer and probe as the internal control group for prevention of false negative results are a primer and probe that are specific to GFP (Green Fluorescence Protein) gene derived from a plant, and do not inhibit Real Time PCR of the HBV-specific primer and probe, and separately form PCR products. Particularly, in the detection of a low level of HBV, the formation of HBV PCR product is not inhibited by the PCR product of the internal control, whereby the false negative results can be effectively prevented, reliability of quantitation result is improved, and the quantitation is also available at a lower detection level. Meanwhile, in the detection of a high level of HBV, formation of the PCR products of the internal control is not inhibited by the PCR products of HBV gene, and thus the test reliability is effectively maintained to reduce the possibility of retest due to inhibition of internal control reaction, thereby providing convenience and high efficiency, as well as allowing quantitative determination in a wide range of levels. The probe base sequence of internal control is SEQ ID NO. 9, as described in Table 2.

In one preferred embodiment, the composition of the present invention may be a composition for the detection and quantification of HBV, which further comprises a buffer solution, DNA polymerase, dNTP and distilled water. In addition, the buffer solution, DNA polymerase, dNTP and distilled water may be any solutions and enzymes that are typically used in the related art, without limitation. Preferably, the composition for the detection of HBV may have the composition of Table 3.

Preferably, the additional enzyme may be HS-Taq. HS-Taq is a modified form of Taq DNA Polymerase, which is activated by heat treatment. A chemical moiety is attached to the enzyme at the active site, which renders the enzyme inactive at room temperature. Thus, during amplification, misprimed primers are not extended. The result is higher specificity and greater yields, when compared to standard DNA polymerases.

The specific composition of the primer, probe, HS-Taq and buffer solution of the present invention is described in the following Table 3.

**[Table 3]**

| | Addition | Added amount (µl) |
|---|---|---|
| Primer | 100 µM BSFOR | 0.1 |
| | 100 µM BSREV | 0.1 |
| | 100 µM BCFOR | 0.1 |
| | 100 µM BCREV | 0.1 |
| | 100 µM ICFOR | 0.1 |
| | 100 µM ICREV | 0.1 |
| Probe | 100 µM BSFAM | 0.02 |
| | 100 µM BCFAM | 0.02 |
| | 100 µM ICCy5 | 0.02 |
| PCR reactant | 2U HS prime Taq | 4.845 |
| | premix | |
| | 1M MGCl₂ | 0.004 |
| | 100mM dUNTP | 0.025 |
| | 2000 U/mL UDG | 0.125 |
| Extracted DNA | | 5 |
| Milli-Q Water | | 4.34 |
| Total | | 20 |

In the present invention, the target amplification reaction may be preferably PCR, and the PCR conditions are described in the following Table 4.

**[Table 4]**

| Reaction Conditions of Real Time PCR | |
|---|---|
| Temperature/Time | Cycle |
| 50°C/2 min | 1 |
| 95°C/10 min | 1 |
| 95°C/15 sec | |
| 57°C/60 sec | 40 |
| 72°C/20 sec | |

In still another embodiment, the present invention relates to a method for detecting HBV gene, comprising the steps of obtaining a sample from a subject; and detecting HBV gene in the biological sample using the composition for the detection of HBV.

The term "biological sample", as used herein, includes tissues, cells, whole blood, serum, plasma, saliva, sputum, cerebrospinal fluid or urine obtained from the individuals with HBV infection, suspected of having HBV infection or vaccinated with HBV vaccine, but is not limited thereto. A sample obtained from the blood of an individual suspected of having HBV infection is preferred.

A method for identifying the presence and genotype of HBV is particularly not limited as long as it employs the aforementioned primers. Examples of such methods include direct identification of HBV DNA using a primer of a specific strand as a probe, Southern blotting, dot blotting, and FISH (filter in situ hybridization). Alternative methods include a method based on amplifying HBV DNA using a pair of primers, genotype-specific polymerase chain reaction (hereinbelow, referred to as PCR), and general-primer PCR. PCR is more preferred, and Real Time PCR is most preferred.

As used herein, the term "polymerase chain reaction (PCR)" is a representative nucleic acid amplification technique (NAT), which enzymatically amplifies a specific DNA region of interest in vitro. The PCR method, which was developed in 1985 by Mullis et al., can amplify any segment of a DNA molecule if its boundary sequences are known. PCR basically consists of three major steps: denaturation, annealing and extension. A specific DNA sequence is amplified while these three steps are repeated. In the first step (denaturation) of PCR, a double-stranded template DNA is denatured into two single strands. In the second step (annealing), primers anneal with the two kinds of single-stranded DNA, in which a sequence desired to be amplified is interposed between the primer binding regions. In the third step, a heat-resistant DNA polymerase extends the primers and synthesizes the complementary strand. This cycle is repeated 25 to 30 times.

Preferably, the composition according to the present invention is used to amplify DNA extracted from the clinical specimen, and the products are subjected to real time PCR. Such real time PCR analysis may be performed by any commercially available Real-time PCR reactor, exemplified by SLAN real time PCR detection system (LG Life Science, Korea), LightCyclerTM (Roche, Germany), ABI PRISMTM 7000/7700 (Applied Biosystems, USA), iCyclerTM (Bio-Rad, USA), Rotor-GeneTM(Corbett, Australia), and OpticonTM (PharmaTech, USA), but is not limited thereto.

Primers are the most important factor determining the reliability of PCR results. Some primer sequences can give rise to non-specific amplification, leading to false results. In this regard, the present invention provides reliable primer pairs. The performance of PCR with the primer pairs of the present invention enables accurate detection of HBV genotypes and sensitive quantitative analysis of very small amounts. Also, when PCR is carried out with the primer pairs of the present invention, consistent results are obtained upon repeated PCR performance. That is, since the primer pairs of the present invention are highly valid and reliable, the results obtained with the present primer pairs are highly reliable.

In still another embodiment, the present invention relates to a kit for the detection and quantification of HBV, comprising the composition for the detection of HBV.

Preferably, the kit can be manufactured by including the primer and probe having the sequences of the present invention, and the internal control primer and probe sequences. The kit may include a buffer, KCl, MgCl₂, and dNTP for HBV detection and quantification. More preferably, the kit having the composition of Table 3 is used to manufacture a kit for the amplification and detection of HBV. In the preferred Example of the present invention, a kit having the above composition was manufactured, thereby detecting and quantifying HBV with high sensitivity and specificity.

### [Mode for Invention]

Hereinafter, the present invention will be described in more detail with reference to Examples. However, these examples are for illustrative purposes only and are not intended to limit the scope of the present invention.

### Example 1. Construction of Primer and Probe

The HBV-specific primer and probe used in the present invention were primer sequences that can amplify only HBV virus gene, confirmed by analyzing the DNA sequence in GenBank (www.ncbi.nlm.nih.gov) managed by National Center for Biotechnology Information (NCBI) of U.S. National Institutes of Health (NIH) using a DNAsis program from Hitachi Software, sequencing the DNA sequence, and then analyzing the DNA sequence again with BLAST (www.ncbi.nlm.nih.gov/BLAST/).

After obtaining the base sequence, the plant-derived gene specific primer and probe that did not competitively inhibit the HBV-specific primer and probe in the composition for HBV detection were determined by using HS-Taq.

### Example 2. Synthesis of Primer

The primers analyzed in Example 1 were synthesized by Metabion (Germany) using a method such as "Synthesis of Oligonucleotide" described in a paragraph 10.42 of Molecular cloning 3rd (Sambrook and Rusell, Cold Spring Harbor Laboratory Press, New York, USA, 2001).

### Examples 3. Extraction of HBV RNA from Clinical Specimen

Serum or EDTA-Plasma was separated from the blood of patients with suspected HBV infection, and extracted using a silica membrane-based spin column such as QIAamp MineElute Virus Spin Column (Qiagen).

### Example 4. Multiplex Real-Time PCR Analysis Using the Primer and Probe

A PCR reaction solution was prepared according to the compositions in Table 3, and PCR was performed under the conditions of Table 4 in a SLAN real time PCR detection system (LG Life Science, Korea). The reaction products were measured in real-time, and the results were analyzed using a SLAN 8.0 program after completing the reaction.

Of the HBV DNA quantification methods using PCR, Cobas Amplicor HBV MonitorTM test (Roche Diagnostics Systems, Meylan, France) has high sensitivity, but a narrow dynamic range of 2.0×10²-2.0×10⁵ copies/mL. Thus, to measure within the linear range of accurate quantification, a sample dilution is required. In addition, a bDNA method is a DNA quantification method based on signal amplification, in which a viral gene present in the serum of a patient is captured using each viral DNA-specific probe and then the captured gene is reacted with a probe and amplifier to quantify DNA. This method has been used in diagnosis of virus infection and evaluation of therapeutic effect for several years. In the present invention, the serum HBV DNA levels of chronic HBV-infected patients in Korea were measured by a real-time PCR which was invented by the present inventors, and compared with Cobas Amplicor HBV MonitorTM test and bDNA method to evaluate its validity.

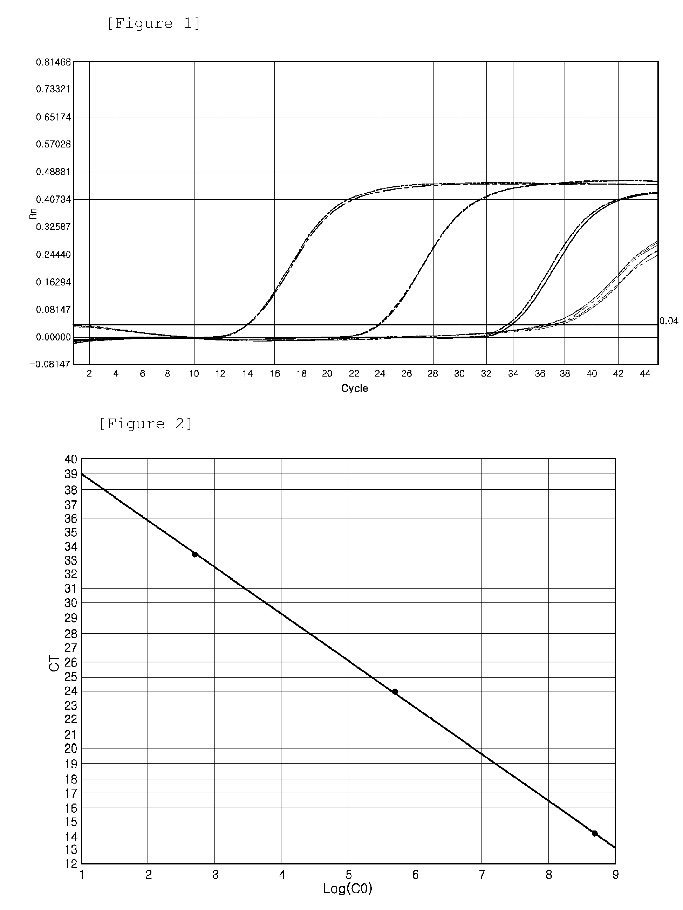

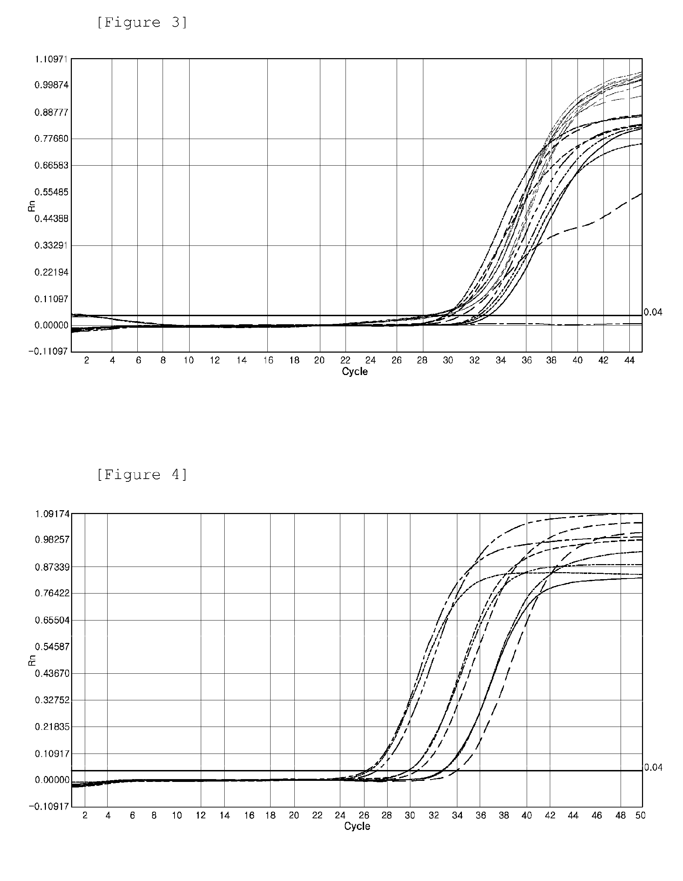

As shown in Table 5 and FIG. 3, the positive predictive value was found to be 100% in the test using the Genotype Panel ((PHD201(M)) provided by BBI, indicating very low risk of false positive and false negative results. In addition, FIG. 1 suggests that a positive detection and quantification can be made without competitive inhibition of the internal control for the HBV-specific primer and probe.

**[Table 5]**

| **Member ID#** | **Origin** | **Genotypes** | **%Identity** | **GI#** | **Roche Cobas Amplicor Monitor (copies/mL)** | **Bayer bDNA (copies/mL)** | **AdvanSure HBV QPCR (copies/mL)** |
|---|---|---|---|---|---|---|---|
| PHD201-01 | US | Negative | N/A | N/A | < 300 | < 2000 | < 58.2 |
| PHD201-02 | US | A | 94 | 416174A | 1.9 X 10⁴ | 4.8 X 10⁴ | 3.2 X 10⁴ |
| | | | 94 | 416076A | | | |
| PHD201-03 | US | A | 97 | 416074A | 1.5 X 10⁴ | 4.2 X 10⁴ | 3.6 X 10⁴ |
| | | | 95 | 416076A | | | |
| PHD201-04 | E Asia | B | 99 | 416078B | 1.9 X 10⁴ | 8.0 X 10³ | 4.7 X 10³ |
| PHD201-05 | E Asia | C | 98 | 452622C | 1.7 X 10⁴ | 1.9 X 10⁴ | 5.6 X 10³ |
| | | | 97 | 416080C | | | |
| | | | 98 | 452641C | | | |
| | | | 98 | 416082C | | | |
| PHD201-06 | Middle East | D | 94 | 416086D | 1.6 X 10⁴ | 9.5 X 10³ | 3.2 X 10³ |
| | | | 95 | 416084D | | | |
| PHD201-07 | US | A | 99 | 416074A | 1.1 X 10⁴ | 1.4 X 10⁴ | 6.5 X 10³ |
| | | | 97 | 416076A | | | |
| PHD201-08 | Africa | E | 99 | X75657 | 2.4 X 10⁴ | 1.8 X 10⁴ | 2.5 X 10⁴ |
| PHD201-09 | South America | F | 99 | X69798 | 1.3 X 10⁴ | 1.5 X 10⁴ | 1.3 X 10⁴ |

### Example 6. Test on HBV detection limit of the kit of the present invention

The kit of the present invention was used to test its detection limit for HBV infection. The test results are shown in the following Table 6.

**[Table 6]**

| Test of Detection Limit | | | |
|---|---|---|---|
| **IU/mL** | **# Tested** | **# Detected** | **% Detected** |
| 10 | 24 | 24 | 100 |
| 6 | 24 | 24 | 100 |
| 5 | 24 | 24 | 100 |
| 3 | 24 | 23 | 96 |
| 2 | 24 | 22 | 92 |
| 1 | 24 | 20 | 83 |
| Probit 95% Hit rate | 2.6 IU/mL (95% confidence limits of 1.6 ∼ 10.4 IU/mL) | | |

To determine the lower limit of detection of HBV, an international standard for HBV, NIBSC 97/746 was applied to compensate the matrix effect for normalization, and then DNA was extracted from 6 different concentrations of HBV sera diluted with Drug Free Serum (Bio-rad), and quantified by AdvanSure HBV Real-Time PCR, followed by Probit analysis and calculation of 95% detection limit. As a result, the 95% detection limit was 2.6 IU/mL.

## Claims

1. A pair of primers complementary to a hepatitis B virus (HBV) genome region, which have the base sequences represented by SEQ ID NOs. 1 and 2, or SEQ ID NOs. 3 and 4.

2. A pair of primers according to claim 1, wherein the hepatitis B virus (HBV) genome region is the S protein region or the pre-core region.

3. A composition for the detection of HBV gene, comprising a pair of primers having the base sequences of SEQ ID NOs. 1 and 2, a pair of primers having the base sequences of SEQ ID NOs. 3 and 4, and probes having the base sequences of SEQ ID NOs. 7 and 8.

4. The composition according to claim 3, wherein the composition further comprises a pair of primers having the base sequences of SEQ ID NOs. 5 and 6 and a probe having the base sequence of SEQ ID NO. 9 as an internal control, and shows no competitive inhibition.

5. The composition according to claim 3, further comprising HS-Taq, a buffer solution, dNTP or distilled water.

6. The composition according to claim 5, comprising each 100 µM of the primers of SEQ ID NOs . 1 to 4, each 100 µM of the probes of SEQ ID NOs. 7 and 8, 2.5U HS-Taq, and 2 mM dUTP.

7. The composition according to claim 3, wherein the probe contains fluorescent molecules at 5' and 3'-ends.

8. The composition according to claim 7, wherein the fluorescent molecule at 5'-end is any one selected from the group consisting of 6-carboxyfluorescein, hexachloro-6-carboxyfluorescein, tetrachloro-6-carboxyfluorescein, FAM (5-carboxy fluorescein), HEX (2',4',5',7'-tetrachloro-6-carboxy-4,7-dichlorofluorescein), and Cy5 (cyanine-5).

9. The composition according to claim 7, wherein the fluorescent molecule at 3'-end is any one selected from the group consisting of 6-carboxytetramethyl-rhodamine, TAMRA (5-Carboxytetramethylrhodamine), and BHQ 1, 2 and 3 (black hole quencher 1, 2, 3).

10. A kit for the detection of HBV, comprising the composition of any one of claims 1 to 9.

11. A method for detecting HBV gene, comprising the steps of obtaining a sample from a subject; and detecting HBV gene in the biological sample using the composition of any one of claims 1 to 9.

12. The method according to claim 11, wherein the detection is performed by polymerase chain reaction.

13. The method according to claim 11, wherein the sample is blood.
